(19) Europäisches Patentamt
European Patent Office
Office européen des brevets

(11) Veröffentlichungsnummer : **0 028 584**
**B1**

(12) # EUROPÄISCHE PATENTSCHRIFT

(45) Veröffentlichungstag der Patentschrift : **16.02.83**

(51) Int. Cl.$^3$ : **C 07 C145/00**, A 01 N 47/24

(21) Anmeldenummer : **80810328.7**

(22) Anmeldetag : **30.10.80**

(54) **Sulfinylamide, Verfahren zu ihrer Herstellung und ihre Verwendung in der Schädlingsbekämpfung.**

(30) Priorität : **05.11.79 CH 9909/79**
**09.09.80 CH 6761/80**

(43) Veröffentlichungstag der Anmeldung : **13.05.81 Patentblatt 81/19**

(45) Bekanntmachung des Hinweises auf die Patenterteilung : **16.02.83 Patentblatt 83/07**

(84) Benannte Vertragsstaaten : **AT BE CH DE FR GB IT LI NL**

(56) Entgegenhaltungen : **DE A 2 655 212**

(73) Patentinhaber : **CIBA-GEIGY AG**
**Patentabteilung Postfach**
**CH-4002 Basel (CH)**

(72) Erfinder : **Kristiansen, Odd, Dr.**
**Delligrabenstrasse 7**
**CH-4313 Möhlin (CH)**
Erfinder : **Drabek, Jozef, Dr.**
**Benkenstrasse 12**
**CH-4104 Oberwil (CH)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 028 584 B1

Sulfinylamide, Verfahren zu ihrer Herstellung und ihre Verwendung in der Schädlingsbekämpfung

Die vorliegende Erfindung betrifft Sulfinylamide, Verfahren zu ihrer Herstellung und ihre Verwendung in der Schädlingsbekämpfung. Die Sulfinylamide haben die Formel

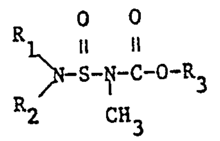

(I)

worin

$R_1$ Wasserstoff oder $C_1$-$C_6$-Alkyl,

$R_2$ $C_1$-$C_6$-Alkyl oder $C_1$-$C_6$-Alkoxy und

$R_3$ unsubstituiertes oder ein oder mehrfach, gleich oder verschieden durch Halogen, $C_1$-$C_6$-Alkyl, $C_1$-$C_6$-Alkoxy, $C_1$-$C_6$-Alkylthio, $C_2$-$C_6$-Alkenyl, $C_2$-$C_6$-Alkinyl, Nitro

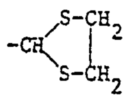

oder

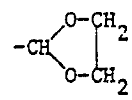

substituiertes Phenyl bedeuten.

In der Deutschen Offenlegungsschrift Nr. 2 655 212 sind insektizide N-Aminothiocarbamate beschrieben. Die erfindungsgemässen Verbindungen der Formel I sind N-Aminosulfinylcarbamate, welche demgegenüber eine bessere systemische und Kontakt-Insektizidwirkung haben.

Unter Halogen bei $R_3$ is Fluor, Chlor, Brom und Jod, insbesondere aber Chlor und Brom, zu verstehen.

Die bei $R_1$, $R_2$ und $R_3$ in Frage kommenden Alkyl-, Alkoxy-, Thioalkyl-, Alkenyl- und Alkinylgruppen können geradkettig oder verzweigt sein. Beispiele solcher Gruppen sind u. a. Methyl, Methoxy, Methylthio, Aethyl, Aethoxy, Aethylthio, n-Propyl, n-Propoxy, n-Propylthio, Isopropyl, Isopropoxy, Isopropylthio, n-, i-, sek.-, tert.-, Butyl, n-Pentyl, n-Hexyl und deren Isomere, Allyl, Propargyl.

Wegen ihrer Wirkung von Bedeutung sind Verbindungen der Formel I, worin

$R_1$ Methyl,

$R_2$ Methyl oder Methoxy und

$R_3$ ein bis dreifach durch $C_1$-$C_6$-Alkyl, $C_1$-$C_6$-Alkoxy, $C_1$-$C_6$-Alkylthio oder

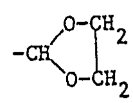

substituiertes Phenyl bedeuten.

Von besonderer Bedeutung sind folgende Verbindungen der Formel I:

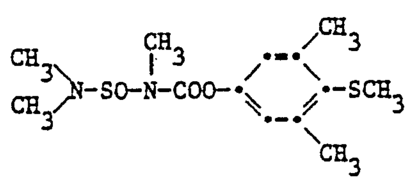

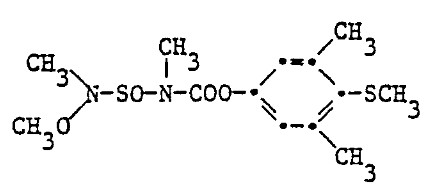

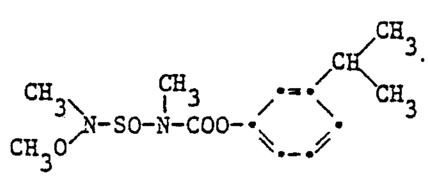

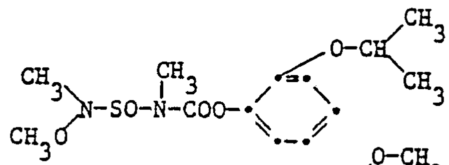

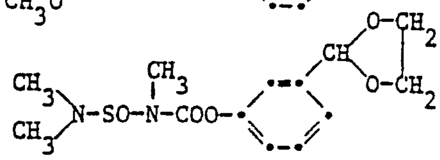

Die Verbindungen der Formel I können nach an sich bekannten Methoden z. B. wie folgt hergestellt werden :

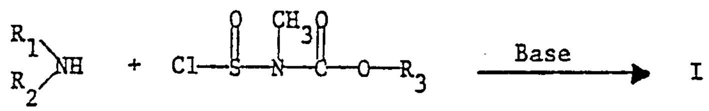


1)

(II) (III)

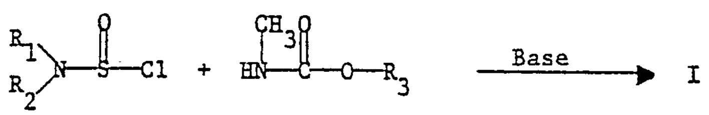


2)

(IV) V

In den Formeln II bis V haben $R_1$, $R_2$ und $R_3$ die gleiche Bedeutung wie für die Formel I. Als geeignete Basen für die Verfahren 1 und 2 kommen Pyridin, Alkylpyridine wie Picoline und Lutidine, tertiäre Amine wie Trialkylamine, ferner Hydroxide, Oxide, Karbonate und Bikarbonate von Alkali- und Erdalkalimetalle, sowie Alkalimetallalkoholate wie z. B. Kalium-t.-butylat und Natriummethylat in Betracht.

Die Verfahren 1 und 2 werden bei einer Reaktionstemperatur zwischen − 20 bis 100 °C meist zwischen − 10 bis 50 °C, bei normalem oder erhöhtem Druck und vorzugsweise in einem inerten Lösungs- oder Verdünnungsmittel durchgeführt. Als Lösungs- oder Verdünnungsmittel eignen sich z. B. Aether oder ätherartige Verbindungen, wie Diäthyläther, Dipropyläther, Dioxan, Dimethoxyäthan und Tetrahydrofuran ; Amide, wie N,N-di-alkylierte Carbonsäureamide ; aliphatische, aromatische sowie halogenierte Kohlenwasserstoffe insbesondere Benzol, Toluol, Xylole, Chloroform und Chlorbenzol ; Nitrile wie Acetonitril ; Dimethylsulfoxid und Ketone, wie Aceton und Methyläthylketon. Die Ausgangsstoffe der Formeln II bis V sind bekannt oder können nach bekannten Methoden hergestellt werden.

Die Verbindungen der Formel I eignen sich zur Bekämpfung von verschiedenartigen tierischen und pflanzlichen Schädlingen, z. B. gegen pflanzenpathogene Nematoden.

Insbesondere eignen sich die Verbindungen der Formel I aber zur Bekämpfung von Insekten, phytopathogenen Milben und von Zecken z. B. der Ordnungen Lepidoptera, Coleoptera, Homoptera, Heteroptera, Diptera, Acarina, Thysanoptera, Orthoptera, Anoplura, Siphonaptera, Mallophaga, Thysanura, Isoptera, Psocoptera und Hymenoptera.

Vor allem eignen sich Verbindungen der Formel I zur Bekämpfung von pflanzenschädigenden Insekten, insbesondere pflanzenschädigenden Frassinsekten, in Zier- und Nutzpflanzen, insbesondere in Baumwollkulturen (z. B. gegen Spodoptera littoralis und Heliothis virescens) und Gemüsekulturen (z. B. gegen Leptinotarsa decemlineata und Myzus persicae).

Wirkstoffe der Formel I zeigen auch eine sehr günstige Wirkung gegen Fliegen, wie z. B. Musca domestica und Mückenlarven.

Die akarizide bzw. insektizide Wirkung lässt sich durch Zusatz von anderen Insektiziden und/oder Akariziden wesentlich verbreitern und an gegebene Umstände anpassen. Als Zusätze eignen sich z. B. org. Phosphorverbindungen; Nitrophenole und deren Derivate ; Formamidine ; Harnstoffe ; pyrethrinartige Verbindungen sowie Karbamate und chlorierte Kohlenwasserstoffe.

Mit besonderem Vorteil werden Verbindungen der Formel I auch mit Substanzen kombiniert, welche einen synergistischen oder verstärkenden Effekt ausüben. Beispiele solcher Verbindungen sind u. a. Piperonylbutoxid, Propinyläther, Propinyloxime, Propinylcarbamate und Propinylphosphonate, 2-(3,4-Methylendioxyphenoxy)-3,6,9-trioxaundecan (Sesamex resp. Sesoxane), S,S,S-Tributylphosphorotrithioate, 1,2-Methylendioxy-4-(2-octylsulfinyl)-propyl)-benzol.

Verbindungen der Formel I können für sich allein oder zusammen mit geeigneten Trägern und/oder Zuschlagstoffen eingesetzt werden. Geeignete Träger- und Zuschlagstoffe können fest oder flüssig sein und entsprechen den in der Formulierungstechnik üblichen Stoffen, wie z. B. natürlichen oder regenerierten Stoffen, Lösungs-, Dispergier-, Netz-, Haft-, Verdickungs-, Binde- und/oder Düngemitteln.

Die Herstellung erfindungsgemässer Mittel erfolgt in an sich bekannter Weise durch inniges Vermischen und/oder Vermahlen der Wirkstoffe der Formel I mit den geeigneten Trägerstoffen, gegebenenfalls unter Zusatz von gegenüber den Wirkstoffen inerten Dispergier- oder Lösungsmitteln. Die Wirkstoffe können in den folgenden Aufarbeitungsformen vorliegen und angewendet werden :

Feste Aufarbeitungsformen : Stäubemittel, Streumittel, Granulate (Umhüllungsgranulate, Imprägnierungsgranulate und Homogranulate) ;

Flüssige Aufarbeitungsformen :

a) in Wasser dispergierbare Wirkstoffkonzentrate : Spritzpulver (wettable powders), Pasten, Emulsionen ;

b) Lösungen.

Der Gehalt an Wirkstoff in den oben beschriebenen Mitteln liegt zwischen 0,1 bis 95 %, dabei ist zu erwähnen, dass bei der Applikation aus dem Flugzeug oder mittels andere geeigneter Applikationsgeräte Konzentrationen bis zu 99,5 % oder sogar reiner Wirkstoff eingesetzt werden können. Die Wirkstoffe der Formel I können beispielsweise wie folgt formuliert werden (Teile bedeuten Gewichtsteile) :

Stäubemittel : Zur Herstellung eines a) 5 %igen und b) 2 %igen Stäubemittels werden die folgenden Stoffe verwendet :

a) 5 Teile Wirkstoff
95 Teile Talkum ;

b) 2 Teile Wirkstoff
1 Teil hochdisperse Kieselsäure
97 Teile Talkum.

Der Wirkstoff wird mit den Trägerstoffen vermischt und vermahlen.

Granulat : Zur Herstellung eines 5 %igen Granulates werden die folgenden Stoffe verwendet :

5 Teile Wirkstoff
0,25 Teile epoxydiertes Pflanzenöl
0,25 Teile Cetylpolyglykoläther
3,50 Teile Polyäthylenglykol
91 Teile Kaolin (Korngrösse 0,3-0,8 mm).

Die Aktivsubstanz wird mit epoxydiertem Pflanzenöl vermischt und mit 6 Teilen Aceton gelöst, hierauf wird Polyäthylenglykol und Cetylpolyglykoläther zugesetzt. Die so erhaltene Lösung wird auf Kaolin aufgesprüht und anschliessend das Aceton im Vakuum eingedampft.

Spritzpulver : Zur Herstellung eines a) 40 %igen, b) und c) 25 %igen, d) 10 %igen Spritzpulvers werden folgende Bestandteile verwendet :

a) 40 Teile Wirkstoff
5 Teile Ligninsulfonsäure-Natriumsalz
1 Teil Dibutylnaphthalinsulfonsäure-Natriumsalz
54 Teile Kieselsäure ;

b) 25 Teile Wirkstoff
4,5 Teile Calcium-Ligninsulfonat
1,9 Teile Champagne-Kreide/Hydroxyäthylcellulose-Gemisch (1 : 1)
1,5 Teile Natrium-dibutyl-naphthalinsulfonat
19,5 Teile Kieselsäure
19,5 Teile Champagne Kreide
28,1 Teile Kaolin ;

c) 25 Teile Wirkstoff
2,5 Teile Isooctylphenoxy-polyäthylen-äthanol
1,7 Teile Champagne-Kreide/Hydroxyäthylcellulose-Gemisch (1 : 1)
8,3 Teile Natriumaluminiumsilikat
16,5 Teile Kieselgur
46 Teile Kaolin ;

d) 10 Teile Wirkstoff
3 Teile Gemisch der Natriumsalze von gesättigten Fettalkoholsulfaten
5 Teile Naphthalinsulfonsäure/Formaldehyd-Kondensat
82 Teile Kaolin

Der Wirkstoff wird in geeigneten Mischern mit dem Zuschlagstoff innig vermischt und auf entsprechenden Mühlen und Walzen vermahlen. Man erhält Spritzpulver, die sich mit Wasser zu Suspensionen jeder gewünschten Konzentration verdünnen lassen.

Emulgierbare Konzentrate : Zur Herstellung eines a) 10 %igen, b) 25 %igen und c) 50 %igen emulgierbaren Konzentrates werden folgende Stoffe verwendet :

a) 10 Teile Wirkstoff
3,4 Teile epoxydiertes Pflanzenöl
3,4 Teile eines Kombinationsemulgators, bestehend aus Fettalkoholpolyglykoläther und Alkylarylsulfonat-Calcium-Salz
40 Teile Dimethylformamid
43,2 Teile Xylol ;

b) 25 Teile Wirkstoff
2,5 Teile epoxydiertes Pflanzenöl
10 Teile eines Alkylarylsulfonat/Fettalkoholpolyglykoläther-Gemisches
5 Teile Dimethylformamid
57,5 Teile Xylol ;

c) 50 Teile Wirkstoff
4,2 Teile Tributylphenol-Polyglykoläther
5,8 Teile Calcium-Dodecylbenzolsulfonat
20 Teile Cyclohexanon
20 Teile Xylol.

Aus solchen Konzentrationen können durch Verdünnen mit Wasser Emulsionen jeder gewünschten Konzentration hergestellt werden.

Sprühmittel : Zur Herstellung eines a) 5 %igen und b) 95 %igen Sprühmittels werden die folgenden Bestandteile verwendet :

a) 5 Teile Wirkstoff
1 Teil epoxydiertes Pflanzenöl
94 Teile Benzin (Siedegrenzen 160-190 °C) ;

b) 95 Teile Wirkstoff
5 Teile epoxydiertes Pflanzenöl.

Beispiel 1 : Herstellung des Karbamates der Formel

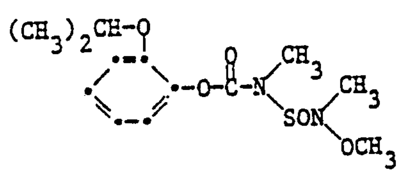

Zu einer Lösung von 9,65 g der Verbindung der Formel

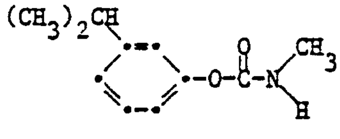

4,8 g Pyridin in 50 ml Tetrahydrofuran werden bei 0-5 °C 6,5 g Thionylchlorid zugetropft. Nach vierstündigem Rühren bei 20 °C werden zuerst 4,8 g Pyridin und anschliessend bei 0-5 °C 3,1 g Methoxymethylamin langsam zugetropft.

Das Gemisch wird vier Stunden bei Raumtemperatur gerührt und mit 2 mal 50 ml Wasser ausgeschüttelt. Nach dem Trocknen, Abdestillieren des Lösungsmittels und der säulenchromatographischen Reinigung ($SiO_2$ ; Chloroform) erhält man die Verbindung der Formel

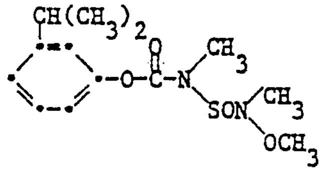

mit einer Refraktion von $n_D^{20°} = 1{,}518\,4$

Auf analoge Weise werden auch folgende Verbindungen hergestellt :

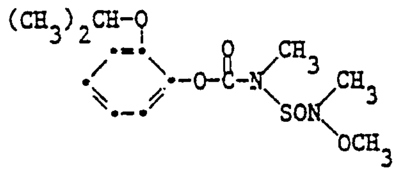

$n_D^{20°} = 1{,}515\,5$

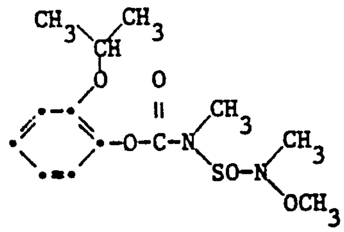

$n_D^{20°}$ : 1,515 0

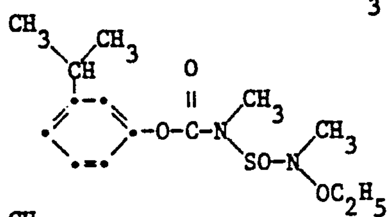

$n_D^{20°}$ : 1,513 1

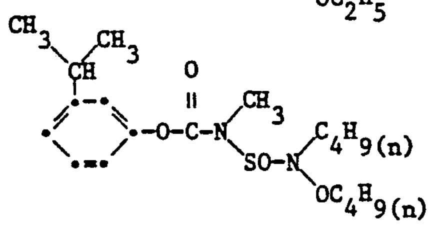

$n_D^{20°}$ : 1,490 2

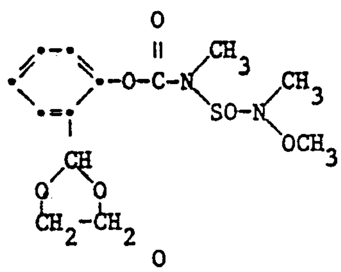

$n_D^{20°}$ : 1,528 7

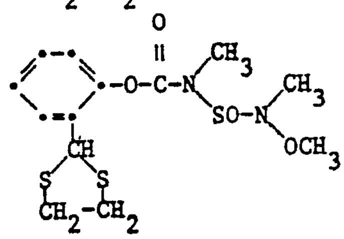

$n_D^{20°}$ : 1,575 0

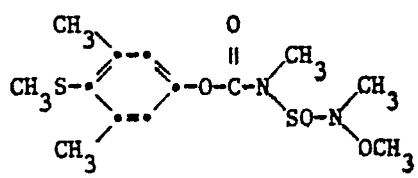

$n_D^{20°}$ : 1,548 6

Beispiel 2 : Insektizide Frassgift-Wirkung : Spodoptera littoralis, Dysdercus fasciatus und Heliothis virescens

Baumwollpflanzen wurden mit einer wässrigen Emulsion enthaltend 0,05 % der zu prüfenden Verbindung (erhalten aus einem 10 %igen emulgierbaren Konzentrat) besprüht.

Nach dem Antrocknen des Belages wurden die Pflanzen je mit Larven der Spezies Spodoptera littoralis (L3-Stadium), Dysdercus fasciatus (L4) oder Heliothis virescens (L3) besetzt. Man verwendete pro Versuchsverbindung und pro Test-Spezies zwei Pflanzen und eine Auswertung der erzielten Abtötungsrate erfolgte nach 2, 4, 24 und 48 Stunden. Der Versuch wurde bei 24 °C und bei 60 % relativer Luftfeuchtigkeit durchgeführt.

Die Verbindungen gemäss Beispiel 1 zeigten im obigen Versuch eine gute Wirkung gegen Larven der Spezies Spodoptera littoralis, Dysdercus fasciatus und Heliothis virescens.

Beispiel 3 : Insektizide Frassgift-Wirkung : Leptinotarsa decemlineata

Bei gleicher Arbeitsweise unter Verwendung von Larven der Spezies Leptinotarsa decemlineata (L3) und Kartoffelpflanzen anstelle von Baumwollpflanzen wurde die im Beispiel 2 beschriebene Versuchsmethode wiederholt.

Die Verbindungen gemäss Beispiel 1 hatten eine gute Wirkung gegen Larven der Spezies Leptinotarsa decemlineata.

Beispiel 4 : Wirkung gegen Dermanyssus gallinae

Je 50 Dermanyssus gallinae werden für kurze Zeit in eine wässrige Emulsion bzw. Lösung enthaltend 0,1 ; 1 ; 10 oder 100 ppm der zu prüfenden Verbindung getaucht. Die in Teströhrchen befindlichen Emulsionen bzw. Lösungen werden dann mit Watte aufgenommen und die benetzten Testtiere in den so kontaminierten Röhrchen belassen.

Eine Auswertung der erzielten Abtötungsrate bei jeder Konzentration erfolgte nach 3 Tagen.

Die Verbindungen gemäss Beispiel 1 zeigten in diesem Versuch eine gute Wirkung gegen Dermanyssus gallinae.

Beispiel 5 : Wirkung gegen Rhipicephalus bursa (Imagines und Larven), Amblyomma hebraeum (♀ Imagines, Nymphen und Larven) und Boophilus microplus (Larven - OP-sensible und OP-tolerant)

Als Testobjekte werden Larven (jeweils ca. 50), Nymphen (jeweils ca. 25) oder Imagines (jeweils ca. 10) von Rhipicephalus bursa, Amblyomma hebraeum und Boophilus microplus verwendet. Die Testtiere werden für kurze Zeit in eine wässrige Emulsion bzw. Lösung enthaltend 0,1 ; 1,0 ; 10 ; 50 oder 100 ppm der zu prüfenden Verbindung getaucht. Die in Teströhrchen befindlichen Emulsionen bzw. Lösungen wurden dann mit Watte aufgenommen und die benetzten Testtiere in den so kontaminierten Röhrchen belassen.

Eine Auswertung der erzielten Abtötungsrate bei jeder Konzentration erfolgte für Larven nach 3 Tagen und für Nymphen und Imagines nach 14 Tagen.

Die Verbindungen gemäss Beispiel 1 zeigten in diesem Versuch eine gute Wirkung gegen Larven, Nymphen und Imagines der Spezies Rhipicephalus bursa und Amblyomma hebraeum sowie gegen Larven (OP-res. und OP-sens.) der Spezies Boophilus microplus.

Beispiel 6 : Wirkung gegen Musca domestica

Je 50 g frisch zubereitetes CSMA-Nährsubstrat für Maden wurde in Becher eingewogen. Von einer 1 Gew.-%igen wässrigen Formulierung des betreffenden Wirkstoffes (dispergierbares Pulver) wurde eine bestimmte Menge auf das in den Bechern befindliche Nährsubstrat pipetiert.

Dann wurden pro Wirkstoff und Konzentration je 25 eintätige Maden von Musca domestica in die das so behandelte Nährsubstrat enthaltenden Becher gegeben. Nachdem sich die Maden verpuppt hatten, wurden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen wurden gezählt (toxischer Einfluss des Wirkstoffes auf die Madenentwicklung). Dann wurde nach 10 Tagen die Anzahl der aus den Puppen geschlüpften Fliegen bestimmt.

Die Verbindungen gemäss Beispiel 1 zeigten gute Wirkung im obigen Test.

Beispiel 7 : Wirkung gegen Aëdes Aegypti

Auf die Oberfläche von 150 ml Wasser, das sich in einem Behälter befindet, wurde so viel einer 0, 1 %igen Emulsions-Zubereitung des Wirkstoffes pipettiert, dass Konzentrationen von je 10,5 und 1 ppm erhalten wurden. Dan wurde der Behälter mit 30-40 2-tägigen Aëdes-Larven beschickt. Nach 1, 2 und 5

Tagen wurde die Mortalität geprüft.

Die Verbindungen gemäss Beispiel 1 zeigten in diesem Test gute Wirkung gegen Aëdes aegypti.

Beispiel 8 : Wirkung gegen Bodennematoden

Zur Prüfung der Wirkung gegen Bodennematoden wurden die Wirkstoffe in der jeweils angegebenen Konzentration in durch Wurzelzellen-Nematoden (Meloidogyne arenaria) infizierte Erde gegeben und innig vermischt. In die so vorbereitete Erde wurden in einer Versuchsreihe unmittelbar danach Tomatensetzlinge gepflanzt und in einer anderen Versuchsreihe nach 8 Tagen Wartezeit Tomaten eingesät.

Zur Beurteilung der nematoziden Wirkung wurden 28 Tagen nach dem Pflanzen bzw. nach der Saat die an den Wurzeln vorhandenen Gallen ausgezählt.

In diesem Test zeigten die Wirkstoffe gemäss Beispiel 1 eine gute Wirkung gegen Meloidogyne arenaria.

**Ansprüche** (für die Vertragsstaaten : BE, CH, DE, FR, GB, IT, LI, NL)

1. Eine Verbindung der Formel

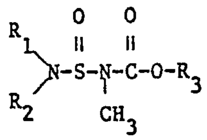

worin

$R_1$ Wasserstoff oder $C_1$-$C_6$-Alkyl,

$R_2$ $C_1$-$C_6$-Alkyl oder $C_1$-$C_6$-Alkoxy und

$R_3$ unsubstituiertes oder ein oder mehrfach, gleich oder verschieden durch Halogen, $C_1$-$C_6$-Alkyl, $C_1$-$C_6$-Alkoxy, $C_1$-$C_6$-Alkylthio, $C_2$-$C_6$-Alkenyl, $C_2$-$C_6$-Alkinyl, Nitro,

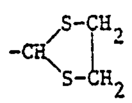 oder 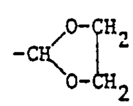

substituiertes Phenyl bedeuten

2. Eine Verbindung gemäss Anspruch 1, worin

$R_1$ Methyl,

$R_2$ Methyl oder Methoxy und

$R_3$ ein bis dreifach durch $C_1$-$C_6$-Alkyl, $C_1$-$C_6$-Alkoxy, $C_1$-$C_6$-Alkylthio oder

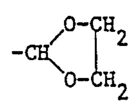

substituiertes Phenyl bedeuten.

3. Die Verbindung gemäss Anspruch 2 der Formel

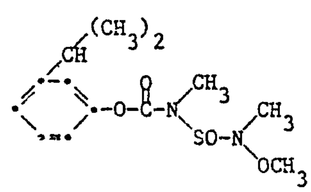

4. Die Verbindung gemäss Anspruch 2 der Formel

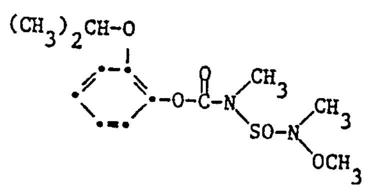

5. Ein Verfahren zur Herstellung von Verbindungen der Formel I, dadurch gekennzeichnet, dass man eine Verbindung der Formel

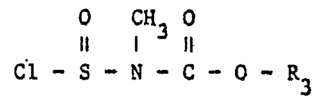

in Gegenwart einer Base mit einer Verbindung der Formel

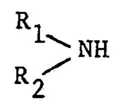

umsetzt, worin $R_1$, $R_2$ und $R_3$ die im Anspruch 1 angegebene Bedeutung haben.

6. Ein Verfahren zur Herstellung von Verbindungen der Formel I, dadurch gekennzeichnet, dass man eine Verbindung der Formel

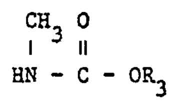

in Gegenwart einer Base mit einer Verbindung der Formel

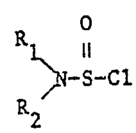

umsetzt, worin $R_1$, $R_2$ und $R_3$ die im Anspruch 1 angegebene Bedeutung haben.

7. Ein Schädlingsbekämpfungsmittel, welches neben geeigneten Träger- und/oder andern Zuschlagstoffen als aktive Komponente eine Verbindung gemäss Anspruch 1 enthält.

8. Verwendung von Verbindungen gemäss Anspruch 1 zur Bekämpfung von tierischen und pflanzlichen Schädlingen.

9. Verwendung gemäss Anspruch 8 zur Bekämpfung von Insekten und Vertretern der Ordnung Akarina.

**Ansprüche** (für den Vertragsstaat AT)

1. Ein Schädlingsbekämpfungsmittel, welches neben geeigneten Träger- und/oder andern Zuschlagstoffen als aktive Komponente eine Verbindung der Formel

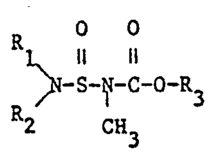

enthält,
worin

$R_1$ Wasserstoff oder $C_1$-$C_6$-Alkyl,

$R_2$ $C_1$-$C_6$-Alkyl oder $C_1$-$C_6$-Alkoxy und

$R_3$ unsubstituiertes oder ein oder mehrfach, gleich oder verschieden durch Halogen, $C_1$-$C_6$-Alkyl, $C_1$-$C_6$-Alkoxy, $C_1$-$C_6$-Alkylthio, $C_2$-$C_6$-Alkenyl, $C_2$-$C_6$-Alkinyl, Nitro

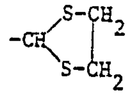

oder

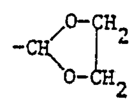

substituiertes Phenyl bedeuten.

2. Ein Mittel gemäss Anspruch 1, welches als aktive Komponente die Verbindung der Formel

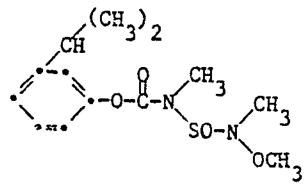

enthält.

3. Ein Mittel gemäss Anspruch 1, welches als aktive Komponente die Verbindung der Formel

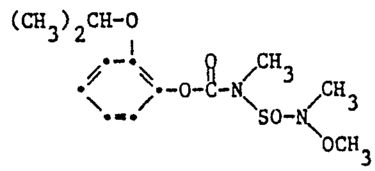

enthält.

**Claims** (for the Contracting States : BE, CH, LI, DE, FR, GB, IT, NL)

1. A compound of the formula

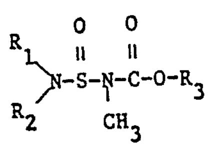

wherein

$R_1$ is hydrogen or $C_1$-$C_6$-alkyl,

$R_2$ is $C_1$-$C_6$-alkyl or $C_1$-$C_6$-alkoxy and

$R_3$ is phenyl which is unsubstituted or mono- or polysubstituted by identical or different substituents selected from the group comprising halogen, $C_1$-$C_6$-alkyl, $C_1$-$C_6$-alkoxy, $C_1$-$C_6$-alkylthio, $C_2$-$C_6$-alkenyl, $C_2$-$C_6$-alkynyl, nitro,

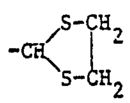 or 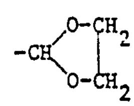

2. A compound according to Claim 1, wherein

$R_1$ is methyl,

$R_2$ is methyl or methoxy, and

$R_3$ is phenyl which is mono- to trisubstituted by $C_1$-$C_6$-alkyl, $C_1$-$C_6$-alkoxy, $C_1$-$C_6$-alkylthio

or 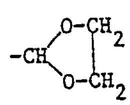

3. The compound according to Claim 2 of the formula

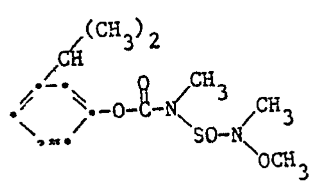

4. The compound according to Claim 2 of the formula

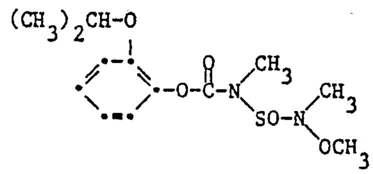

5. A process for producing compounds of the formula I, which process comprises reacting a compound of the formula

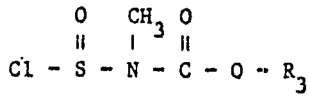

in the presence of a base, with a compound of the formula

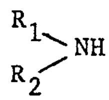

wherein $R_1$, $R_2$ and $R_3$ have the meanings given in Claim 1.

6. A process for producing compounds of the formula I, which process comprises reacting a compound of the formula

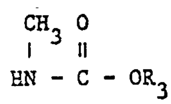

in the presence of a base, with a compound of the formula

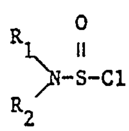

wherein $R_1$, $R_2$ and $R_3$ have the meanings given in Claim 1.

7. A pesticidal composition which comprises a compound according to Claim 1 as active ingredient, and suitable carriers and/or other additives.

8. A method of combating animal and plant pests at a locus, which method comprises applying to the locus a compound as claimed in Claim 1.

9. A method according to Claim 8 for combating insects, and members of the order Acarina.

**Claims** (for the Contracting State AT)

1. A pesticidal composition which contains, besides suitable carriers and/or other additives, as active ingredient a compound of the formula

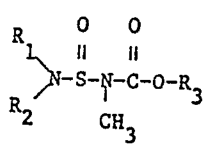

wherein

$R_1$ is hydrogen or $C_1$-$C_6$-alkyl,

$R_2$ is $C_1$-$C_6$-alkyl or $C_1$-$C_6$-alkoxy, and

$R_3$ is phenyl which is unsubstituted or mono- or polysubstituted by identical or different substituents selected from the group comprising halogen, $C_1$-$C_6$-alkyl, $C_1$-$C_6$-alkoxy, $C_1$-$C_6$-alkylthio, $C_2$-$C_6$-alkenyl, $C_2$-$C_6$-alkynyl, nitro,

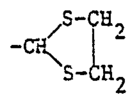

or

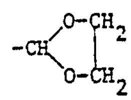

2. A composition according to Claim 1, which contains as active ingredient the compound of the formula

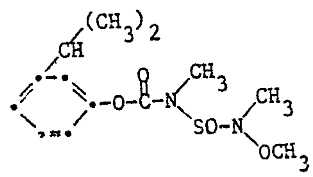

3. A composition according to Claim 1, which contains as active ingredient the compound of the formula

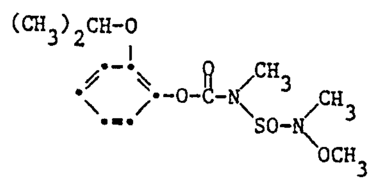

**Revendications** (pour les Etats contractants : BE, CH, LI, DE, FR, GB, IT, NL)

1. Composé de formule

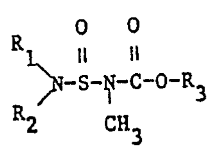

où

$R_1$ représente un hydrogène ou un alcoyle en $C_1$ à $C_6$,

$R_2$ un alcoyle en $C_1$ à $C_6$ ou un alcoxy en $C_1$ à $C_6$ et

$R_3$ un phényle non substitué ou mono- ou poly-substitué, par des substituants semblables ou différents, qui sont un halogène, un alcoyle en $C_1$ à $C_6$, un alcoxy en $C_1$ à $C_6$, un alcoylthio en $C_1$ à $C_6$, un alcényle en $C_2$ à $C_6$, un alcynyle en $C_2$ à $C_6$, un nitro,

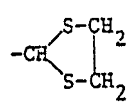

ou

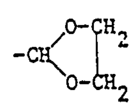

2. Composé selon la revendication 1, où

$R_1$ représente un méthyle,

$R_2$ un méthyle ou un méthoxy et

$R_3$ un phényle mono- à tri-substitué par un alcoyle en $C_1$ à $C_6$, un alcoxy en $C_1$ à $C_6$, un alcoylthio en $C_1$ à $C_6$ ou

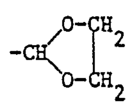

3. Composé selon la revendication 2 de formule

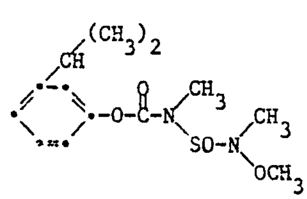

4. Composé selon la revendication 2 de formule

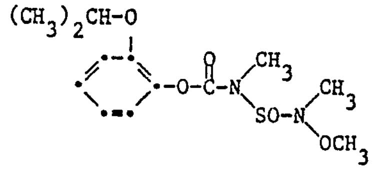

5. Procédé de préparation de composés de formule I, caractérisé en ce qu'on fait réagir un composé de formule

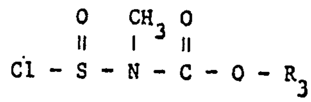

en présence d'une base avec un composé de formule

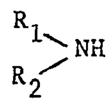

où $R_1$, $R_2$ et $R_3$ ont la signification donnée dans la revendication 1.

6. Procédé de préparation de composés de formule I, caractérisé en ce qu'on fait réagir un composé de formule

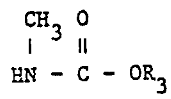

en présence d'une base avec un composé de formule

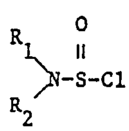

où $R_1$, $R_2$ et $R_3$ ont la signification donnée dans la revendication 1.

7. Agent de lutte anti-parasitaire contenant, outre des supports et/ou autres additifs appropriés, comme composant actif un composé selon la revendication 1.

8. Application de composés selon la revendication 1 à la lutte contre les parasites animaux ou végétaux.

9. Application selon la revendication 8 à la lutte contre les insectes et les représentants de l'ordre des acariens.

**Revendications** (pour l'Etat contractant AT)

1. Agent de lutte anti-parasitaire, contenant, outre des supports et/ou autres additifs appropriés, comme composant actif un composé de formule

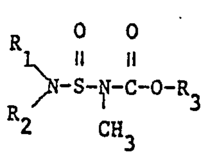

où

$R_1$ représente un hydrogène ou un alcoyle en $C_1$ à $C_6$,

$R_2$ un alcoyle en $C_1$ à $C_6$ ou un alcoxy en $C_1$ à $C_6$ et

$R_3$ un phényle non substitué ou mono- ou poly-substitué par des substituants semblables ou

différents qui sont un halogène, un alcoyle en $C_1$ à $C_6$, un alcoxy en $C_1$ à $C_6$, un alcoylthio en $C_1$ à $C_6$, un alcényle en $C_2$ à $C_6$, un alcynyle en $C_2$ à $C_6$, un nitro,

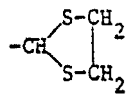

ou

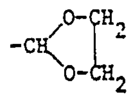

2. Agent selon la revendication 1 contenant comme composant actif le composé de formule

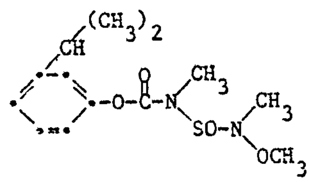

3. Agent selon la revendication 1 contenant comme composant actif le composé de formule